# EUROPÄISCHE PATENTSCHRIFT

(11) **EP 3 355 988 B1**
(45) Veröffentlichungstag und Bekanntmachung des Hinweises auf die Patenterteilung: **27.02.2019**
(21) Anmeldenummer: 16775227.8
(22) Anmeldetag: 29.09.2016
(51) Int. Cl.: A61N 2/00

(54) **MAGNETISCHE STIMULATIONSVORRICHTUNG**
MAGNETIC STIMULATION DEVICE
DISPOSITIF DE STIMULATION MAGNÉTIQUE

(30) Priorität: 02.10.2015 AT 508392015
(43) Veröffentlichungstag der Anmeldung: 08.08.2018
(73) Patentinhaber: Pontemed AG, 9053 Teufen (CH)
(72) Erfinder: MAYR, Winfried, 2340 Mödling (AT)
(74) Vertreter: Sonn & Partner Patentanwälte
(86) Internationale Anmeldenummer: PCT/EP2016/073281
(87) Internationale Veröffentlichungsnummer: WO 2017/055465

(56) Entgegenhaltungen:
- WO-A1-2014/164926
- US-A1- 2006 187 607
- US-A1- 2012 086 449
- US-A1- 2013 150 653

## Beschreibung

Die Erfindung betrifft eine Vorrichtung zur magnetischen Stimulation von Regionen eines menschlichen oder tierischen Körpers, mit zumindest einer Magnetspule, welche mit einem Stimulator verbunden ist, der einen Leistungsteil zur Erzeugung an die zumindest eine Magnetspule anzulegende elektrischer Impulse aufweist, sodass das in der zumindest einen Magnetspule erzeugte Magnetfeld in der Körperregion induzierbar ist, wobei eine Detektionseinheit zur Detektion von Metallelementen innerhalb der Körperregion, in welcher das Magnetfeld induziert wird, vorgesehen ist, und die Detektionseinheit mit einer Anzeigeeinheit und dem Simulator verbunden ist.

Im Gegensatz zur funktionellen Elektrostimulation (FES), bei der ein Muskel oder Nerv zur Durchführung einer Muskelkontraktion oder zur Beeinflussung anderer Nervenfunktionen über kontaktierende Elektroden elektrisch stimuliert wird, um bestimmte physiologische Vorgänge zu unterstützen bzw. zu ersetzen, wird bei der funktionellen Magnetstimulation (FMS) eine Nervenaktivierung, die beispielsweise zu einer Muskelkontraktion führen kann, durch entsprechende Magnetfelder berührungslos ausgelöst.

Die funktionelle Magnetstimulation hat gegenüber funktioneller Elektrostimulation mit an der Hautoberfläche angeordneten Elektroden den wesentlichen Vorteil, dass in der Haut liegende Schmerzsensoren wesentlich geringer aktiviert werden und die Anwendung bei vergleichbarer neuromuskulärer Aktivierung als wesentlich angenehmer empfunden wird. Dies beruht auf der Tatsache, dass die Schmerzsensoren in im Vergleich zu tieferliegenden Gewebeanteilen höherohmigen Gewebeschichten liegen. Der Stromfluss bei elektrischer Stimulation bewirkt daher relativ hohe elektrische Feldstärken besonders im Bereich der Schmerzsensorik, während die wirkungsrelevanten induzierten Wirbelströme bei magnetischer Stimulation im niederohmigen tieferliegenden Gewebe wesentlich stärker ausgeprägt sind als in oberflächennahen höherohmigen Gewebe.

Weiters ist bei der funktionellen Magnetstimulation der Aufwand und das Risiko durch den Wegfall der häufig notwendigen Implantation von Nerven- oder Muskelelektroden bei der funktionellen Elektrostimulation wesentlich niedriger und die Akzeptanz höher. Demgegenüber ist jedoch die gezielte Stimulation bestimmter Nerven oder Muskeln über das Magnetfeld schwieriger als bei der direkten elektrischen Stimulation mit Hilfe von Hautelektroden oder implantierten Elektroden. Insbesondere ist es bei der Stimulation tieferliegender Regionen sehr schwierig bestimmte Punkte, sogenannte motorische Reizpunkte oder Motorpoints mit dem Magnetfeld zu erreichen und beispielsweise die Kontraktion der gewünschten Muskeln zu erzielen.

Einen weiteren Nachteil bei der funktionellen Magnetstimulation stellen Metallelemente innerhalb der zu stimulierenden Körperregion dar, in welche unzulässig hohe Ströme induziert und eine gefährliche Erwärmung der Metallelemente und des umliegenden Gewebes folgen kann. Als Beispiele derartiger Metallelemente, werden Implantate, künstliche Gelenke oder dgl. erwähnt.

Ein Beispiel für eine Vorrichtung zur magnetischen Stimulation wird in der WO 2009/126117 A1 beschrieben. Dabei wird mit Hilfe einer Magnetspule ein Magnetfeld in tiefere Gewebeschichten induziert, wodurch eine Depolarisation neuronaler Zellen resultiert, welche zu Muskelkontraktionen bestimmter Muskeln in bestimmten Körperregionen führen.

Ein weiteres Verfahren und eine Vorrichtung zur neuromagnetischen Stimulation ist aus der EP 0 617 982 A1 bekannt geworden, wobei dem Magnetfeld ein fokussierter Ultraschallstrahl überlagert wird, wodurch eine genauere räumliche Stimulation ermöglicht werden soll.

Ein Verfahren und eine Vorrichtung für das Beckenbodentraining mit Hilfe magnetischer Stimulation ist beispielsweise aus der DE 10 2012 012 149 A1 bekannt geworden. Dabei wird zusätzlich zur Magnetstimulation dem Gewebe noch Sauerstoff und bzw. oder Ozon zugeführt um das Training und den Aufbau der Muskulatur noch weiter zu unterstützen.

Die US 2013/150653 A1 beschreibt eine Vorrichtung zur magnetischen Stimulation der gegenständlichen Art, wobei eine Detektionseinheit zur Detektion von Metallelementen innerhalb der behandelten Körperregion, in welcher das Magnetfeld induziert wird, in Form eigener Messspulen offenbart wird.

Die Aufgabe der Erfindung besteht daher in der Schaffung einer oben genannten magnetischen Stimulationsvorrichtung, durch welche eine gefährliche Erwärmung von Metallelementen innerhalb der Körperregion, in welcher das Magnetfeld induziert wird, wirkungsvoll verhindert werden kann. Nachteile bekannter Stimulationsvorrichtungen sollen vermieden oder zumindest reduziert werden.

Gelöst wird die erfindungsgemäße Aufgabe dadurch, dass eine Messeinrichtung zur Erfassung der von der zumindest einen Magnetspule aufgenommenen elektrischen Leistung als Reaktion auf ein in die Magnetspule eingespeistes Testsignal durch Messung von Betrag und Phase des Stroms und der Spannung an der Magnetspule und eine Vergleichseinrichtung zum Vergleich der aufgenommenen elektrischen Leistung mit einem vorgegebenen Grenzwert, der bei einer vorausgehenden Kalibration eingestellt wird, vorgesehen ist, und die Vergleichseinrichtung dazu ausgebildet ist, im Falle der Überschreitung des vorgegebenen Grenzwerts den Stimulator automatisch abzuschalten oder die Leistung des Stimulators bzw. des Leistungsteils des Stimulators zu reduzieren und dass zumindest eine weitere Detektionseinheit vorgesehen ist, welche durch zumindest einen Ultraschallsender und zumindest einem Ultraschallempfänger, eine Auswerteeiheit und bzw. oder durch zumindest zwei Hautelektroden und eine Einrichtung zur Messung der Gewebeimpedanz der jeweiligen Körperregion gebildet ist,. Die Detektion von Metallelementen über die von der zumindest einen Magnetspule aufgenommenen elektrischen Leistung stellt eine elegante integrierte Lösung einer Detektionseinheit dar, bei der wenig zusätzliche Bauteile erforderlich sind, da die Detektion der Metallelemente über die Rückkopplung zur Magnetspule der Stimulationsvorrichtung erfasst wird. Die Erfassung der von der Magnetspule aufgenommenen elektrischen Leistung und der Vergleich mit entsprechenden Grenzwerten, kann relativ einfach und kostengünstig in einem üblicherweise ohnedies vorhandenen Mikroprozessor oder dergl. in der magnetischen Stimulationsvorrichtung implementiert werden. Durch die in der magnetischen Stimulationsvorrichtung enthaltenen Detektionseinheit kann somit das Vorhandensein von Metallelementen innerhalb der zu behandelnden Körperregion zumindest angezeigt werden und beispielsweise eine Änderung der Position der Stimulationsvorrichtung vorgenommen werden, bevor mit der Stimulation begonnen wird. Somit kann durch das Detektieren des Vorhandenseins von Metallelementen eine unzulässige und gefährliche Erwärmung der Metallelemente bzw. Implantate verhindert werden. Somit kann das nicht unerhebliche Risiko von potentiellen Gewebe schädigenden Erwärmungen oder auch Beschädigung medizinischer Implantate weitestgehend reduziert werden. Bisher war dies nur unter sorgfältiger und umfassender begleitender medizinischer Abklärung bzw. das Durchführen von Röntgenaufnahmen vor der Stimulation vermeidbar.

Dadurch, dass die Detektionseinheit mit dem Stimulator verbunden ist, kann beim Detektieren von Metallelementen innerhalb der Körperregion, in welcher das Magnetfeld induziert werden soll, automatisch der Stimulator bzw. dessen Leistungsteil deaktiviert oder die Leistung reduziert werden, um eine unzulässige Erwärmung der Metallelemente bzw. Implantate sicher verhindern zu können.

Diese Variante stellt eine elegante integrierte Lösung einer Detektionseinheit dar, bei der wenig zusätzliche Bauteile erforderlich sind, da die Detektion der Metallelemente über die Rückkopplung zur Magnetspule der Stimulationsvorrichtung erfasst wird. Die Erfassung der von der Magnetspule aufgenommenen elektrischen Leistung und der Vergleich mit entsprechenden Grenzwerten, kann relativ einfach und kostengünstig in einem üblicherweise ohnedies vorhandenen Mikroprozessor oder dgl. in die magnetische Stimulationsvorrichtung implementiert werden.

Zusätzlich zu der indirekten Detektion von Metallelementen über die Leistungsaufnahme der zumindest einen Magnetspule ist zumindest eine weitere Detektionseinheit vorgesehen, welche durch zumindest einen Ultraschallsender und zumindest einen Ultraschallempfänger und eine Auswerteeinheit gebildet sein kann. Eine derartige Realisierung der weiteren Detektionseinheit ist zwar durch einen höheren Hardware-technischen Aufwand gekennzeichnet, kann jedoch entsprechende Metallelemente im Körper mit höherer Genauigkeit feststellen.

Alternativ oder zusätzlich kann die weitere Detektionseinheit auch durch zumindest zwei Hautelektroden und eine Einrichtung zur Messung der Gewebeimpedanz der jeweiligen Körperregion gebildet sein. Durch das Einprägen eines bestimmten Stromes bzw. einer bestimmten Spannung über die zumindest zwei Hautelektroden, beispielsweise Klebeelektroden, und die Berechnung der resultierenden Gewebeimpedanz kann ebenfalls relativ zuverlässig und mit geringem technischen Aufwand das Vorhandensein von Implantaten oder dgl. im Körper noch besser festgestellt werden.

Schließlich kann die weitere Detektionseinheit auch durch zumindest eine Messspule gebildet sein. Durch eine von der Stimulationsspule unterschiedliche Messspule, welche eine von der Stimulationspule unterschiedliche Windungsanzahl und ein unterschiedliches Frequenzverhalten aufweist, können in Art eines Metalldetektors ebenfalls Implantate oder dgl. in der zu stimulierenden Körperregion vor Durchführung der Stimulation erfasst werden. Um eine Induktion des Magnetfelds der zumindest einen Magnetspule in der zumindest eine Messspule und entsprechend hohe schädigende Ströme zu vermeiden, kann die Messung auch vor Durchführung der Stimulation vorgenommen und danach die Messspule deaktiviert werden.

Die Anzeigeeinheit kann durch eine optische Anzeigeeinheit gebildet sein. Eine derartige optische Anzeigeeinheit kann im einfachsten Fall durch zumindest eine Leuchtdiode oder dgl. realisiert werden oder auch durch eine aufwändigere Anzeigeeinheit, wie z.B. ein LCD-Paneel, gebildet sein. Über die optische Anzeigeeinheit wird dem jeweiligen Bedienungspersonal die Anwesenheit von Metallelementen innerhalb der zu stimulierenden Körperregion des Patienten angezeigt, wodurch vor Durchführung der Stimulation eine Veränderung der Lage der zumindest einen Magnetspule vorgenommen werden kann.

Ebenso kann die Anzeigeeinheit durch eine akustische Anzeigeeinheit gebildet sein. Für sich oder zusätzlich zur optischen Anzeigeeinheit kann eine solche akustische Anzeigeeinheit dem Benutzer durch Abgabe von akustischen Signalen die Anwesenheit von Metallelementen in der zu stimulierenden Körperregion signalisieren.

Schließlich kann die Anzeigeeinheit auch durch einen mechanischen Schwingungserzeuger gebildet werden, um dem Benutzer oder auch dem Patienten durch entsprechende Vibrationen anzuzeigen, dass eine Repositionierung der zumindest einen Magnetspule vorgenommen werden soll.

Vorteilhafterweise ist die zumindest eine Magnetspule in einem Gehäuse angeordnet. Durch ein entsprechendes Gehäuse wird einerseits die Magnetspule sicher elektrisch gegen Berührung isoliert und vor Beschädigung geschützt und andererseits das Anordnen der Magnetspule an der jeweiligen Körperregion erleichtert und auch ein Reinigen bzw. Desinfizieren der Bestandteile der Stimulationsvorrichtung erleichtert.

Auch die Detektionseinheit und allenfalls die zumindest eine weitere Detektionseinheit kann im Gehäuse angeordnet sein. Dadurch wird eine kompaktere Bauweise erzielt und auch sichergestellt, dass die Metallelemente auch wirklich in der Region detektiert werden, in welcher das Magnetfeld zur Stimulation wirkt.

Die Erfindung wird anhand der beigefügten Zeichnungen näher erläutert. Darin zeigen:
- Fig. 1: ein Blockschaltbild einen magnetischen Stimulationsvorrichtung mit einer Detektionseinheit in allgemeiner Form;
- Fig. 2: ein Blockschaltbild der magnetischen Stimulationsvorrichtung mit der erfindungsgemäßen Variante der Detektionseinheit, welche aus der elektrischen Leistungsaufnahme der Magnetspule auf vorhandene Metallelemente in der Körperregion rückschließt;
- Fig. 3: ein Blockschaltbild der magnetischen Stimulationsvorrichtung mit einer Variante einer weiteren Detektionseinheit mit Ultraschallsendern und -empfängern;
- Fig. 4: ein Blockschaltbild der magnetischen Stimulationsvorrichtung mit einer Variante einer weiteren Detektionseinheit mit Hautelektroden und Impedanzmessung; und
- Fig. 5: ein Blockschaltbild der magnetischen Stimulationsvorrichtung mit eine Variante einer weiteren Detektionseinheit in Form einer Messspule.

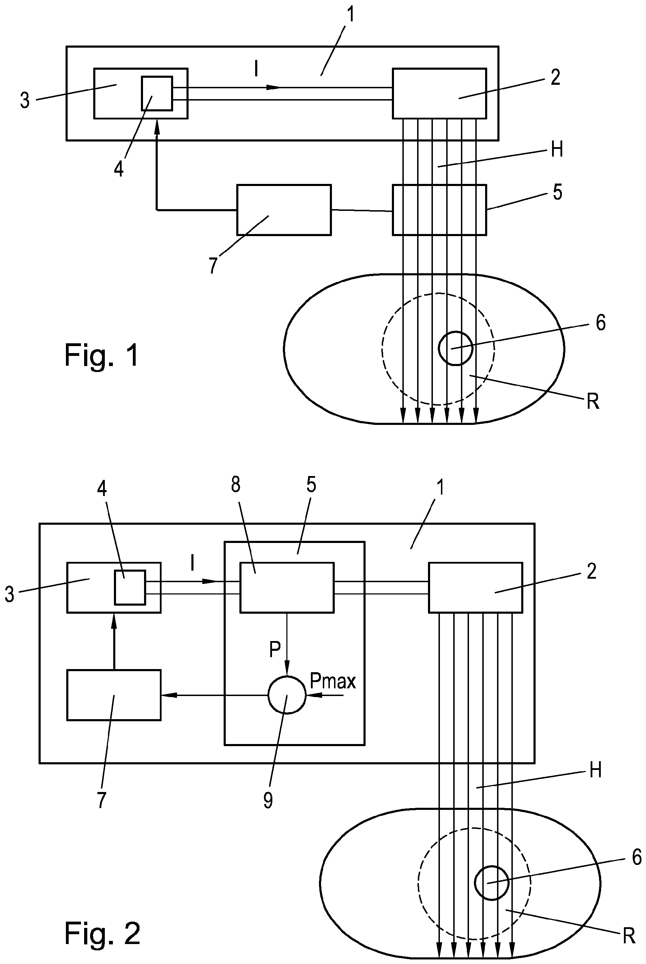

Fig. 1 zeigt ein Blockschaltbild eine magnetischen Stimulationsvorrichtung 1 mit einer Detektionseinheit 5 in allgemeiner Form. Die Vorrichtung 1 zur magnetischen Stimulation von Regionen R eines menschlichen oder tierischen Körpers beinhaltet zumindest eine Magnetspule 2, welche mit einem Stimulator 3 verbunden ist, der einen Leistungsteil 4 zur Erzeugung elektrischer Impulse I, welche an die zumindest eine Magnetspule 2 angelegt werden, aufweist. Durch die elektrischen Impulse I wird in der zumindest einen Magnetspule 2 ein Magnetfeld H erzeugt, welches in der Körperregion R induziert wird und dort an gewünschten Punkten, z.B. an sogenannten Motorpoints, Wirkungen hervorruft, die zu Muskelkontraktionen der gewünschten Körperregion R führen können oder andere Nervenfunktionen beeinflussen. Unter den Begriff Impulse fallen sowohl Rechteckimulse als auch andere Stromformen, durch welche Wechselfelder in der Magnetspule 2 erzeugt werden. Wenn in der jeweiligen Körperregion R, in welcher das Magnetfeld H der zumindest einen Magnetspule 2 induziert wird, Metallelemente 6, wie z.B. Implantate, Knochenschrauben oder dgl. angeordnet sind, kann das Magnetfeld H der zumindest einen Magnetspule 2 in diesen Metallelementen 6 Wirbelströme induzieren, die zu einer gefährlichen Erwärmung der Metallelemente 6 führen können. Bereits bei einer Überschreitung von ca. 43 °C kann es zu einer Denaturierung der Eiweißanteile im umliegenden Gewebe der Körperregion R und dramatischen Gewebeschäden kommen. Daher ist es besonders wichtig, vor der Aktivierung der Stimulationsvorrichtung 1 abzuklären, ob in der zu stimulierenden Körperregion R Metallelemente 6 vorhanden sind. Dies wird gemäß der vorliegenden Erfindung mit einer Detektionseinheit 5 durchgeführt, welche das Vorhandensein von Metallelementen 6 feststellt und an einer Anzeigeeinheit 7 optisch oder akustisch darstellt bzw. wiedergibt sowie eine direkte Einflussnahme auf den Stimulator 3 bzw. den Leistungsteil 4 und somit eine Regelung der Stimulationsimpulse I für die zumindest eine Magnetspule 2 bewirkt, was durch die Verbindungslinie zwischen Anzeigeeinheit 7 und Stimulator 3 dargestellt ist. Durch die Detektionseinheit 5 kann auch ohne vorherige Abklärung mit dem Patienten bzw. ohne die Durchführung von Röntgenaufnahmen eine magnetische Stimulation mit hoher Sicherheit vorgenommen werden, ohne die Gefahr von Gewebeschäden in Kauf nehmen zu müssen.

Fig. 2 zeigt ein Blockschaltbild der magnetischen Stimulationsvorrichtung 1 mit der erfindungsgemäßen Variante der Detektionseinheit 5, welche aus der elektrischen Leistungsaufnahme der Magnetspule 2 auf vorhandene Metallelemente 6 in der Körperregion R rückschließt. Dabei wird durch eine Messeinrichtung 8 die von der zumindest einen Magnetspule 2 aufgenommene elektrische Leistung P erfasst und einer Vergleichseinrichtung 9 zugeführt, in der ein Vergleich der gemessenen elektrischen Leistung P mit einem vorgegebenen Grenzwert Pₘₐₓ durchführt. Überschreitet die durch die zumindest eine Magnetspule 2 aufgenommene elektrische Leistung P den vorgegebenen Grenzwert Pₘₐₓ, so führt dies zu einer optischen oder akustischen Wiedergabe an der Anzeigeeinheit 7 oder zu einer automatischen Abschaltung oder Leistungsreduktion des Stimulators 3 bzw. des Leistungsteils 4 des Stimulators 3.

Die Detektion von Metallelementen 6 über die Leistungsaufnahme der Magnetspule 2 kann durch Erfassung der Reaktion der Magnetspule 2 auf die Stimulationsimpulse oder entsprechende Testsignale durchgeführt werden. Als Stimulationsimpulse werden üblicherweise einzelne Perioden von sinusförmigen Signalen verwendet. Als Testsignale sind insbesondere sinusförmige Signale niedrigerer Amplitude mit sich ändernder Frequenz besonders geeignet. Als Reaktion werden Strom und Spannung in Betrag und Phase an der Magnetspule 2 gemessen und somit die komplexe Impedanz der Magnetspule 2 ermittelt. Durch das Vorhandensein von Metallteilen innerhalb des magnetischen Feldes der Magnetspule 2 ändert sich diese Impedanz, was mit der erfindungsgemäßen Methode erfasst wird. Der Messung der Reaktion der Magnetspule 2 auf das Stimulationssignal oder ein Testsignal, kann auch eine Kalibration vorausgehen, bei welcher sich im Bereich der Magnetspule 2 kein Gegenstand befindet. Über diese Kalibration kann der Grenzwert besser eingestellt werden, ab dem eine Abschaltung oder Leistungsreduktion des Stimulators 3 bzw. des Leistungsteils 4 des Stimulators 3 erfolgen soll. Anstelle eines sich in der Frequenz ändernden Testsignals kann auch ein rechteckiger Impuls verwendet werden, der im Spektrum entsprechend viele Frequenzen aufweist.

In dieser Ausführungsvariante sind die Detektionseinheit 5 und die Anzeigeeinheit 7 in die Stimulationsvorrichtung 1 integriert.

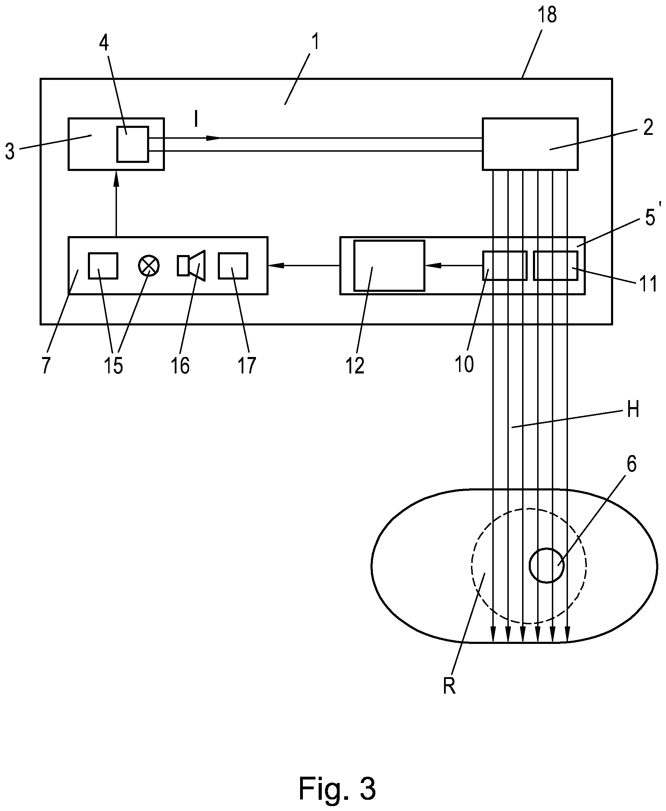

Fig. 3 zeigt ein Blockschaltbild der magnetischen Stimulationsvorrichtung 1 mit einer Variante einer weiteren Detektionseinheit 5' mit Ultraschallsendern 10 und -empfängern 11. Durch eine geeignete Anordnung der Ultraschallsender 10 und Ultraschallempfänger 11 und eine entsprechende Auswertung in einer Auswerteeinheit 12, kann das Vorhandensein von Metallelementen 6 innerhalb der Körperregion R, in welcher das Magnetfeld H der zumindest einen Magnetspule 2 induziert werden soll, noch besser festgestellt werden. Die Auswerteeinheit 12 ist mit der Anzeigeeinheit 7 verbunden um das Ergebnis der Detektion an der Anzeigeeinheit 7 darstellen zu können.

Die Anzeigeeinheit 7 kann durch eine optische Anzeigeeinheit 15, beispielsweise Leuchtdioden oder einen LCD-Bildschirm oder dgl. gebildet sein. Weiters durch eine akustische Anzeigeeinheit 16 und bzw. oder durch einen mechanischen Schwingungserzeuger 17 realisiert werden, der beispielsweise auch in einem Handgriff für die Positionierung der Magnetspule 2 untergebracht sein kann (nicht dargestellt).

Die zumindest eine Magnetspule 2 zur Durchführung der funktionellen Magnetstimulation, kann in einem Gehäuse 18 angeordnet werden, in dem vorzugsweise auch die Detektionseinheit 5 und die weitere Detektionseinheit 5' und die Anzeigeeinheit 7 untergebracht wird. Durch die Anordnung in einem gemeinsamen Gehäuse 18 wird die Reinigung der Vorrichtung 1 erleichtert und auch die Zuordnung der Stimulationselemente und der Detektionselemente sichergestellt.

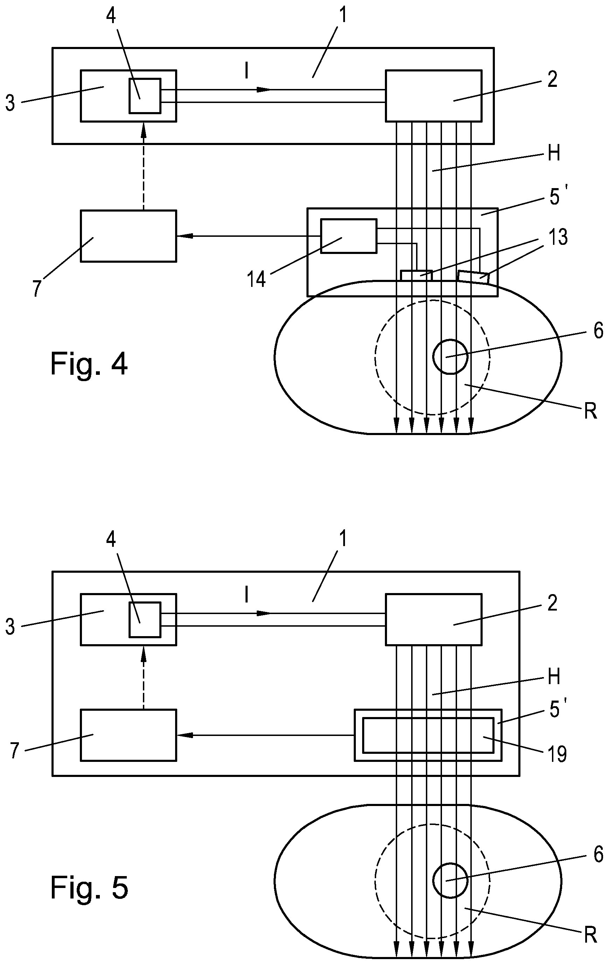

In Fig. 4 ist ein Blockschaltbild der magnetischen Stimulationsvorrichtung 1 mit einer Variante einer weiteren Detektionseinheit 5' mit Hautelektroden 13 und Messung der Gewebeimpedanz Z dargestellt. Diese allfällige zusätzliche Ausführungsform der weiteren Detektionseinheit 5', ist durch eine Strom oder Spannungseinprägung über die Hautelektroden 13 gekennzeichnet und eine Messeinrichtung 14, welche die Gewebeimpedanz Z der jeweiligen Körperregion R misst. Wenn die Gewebeimpedanz Z bestimmte Grenzwerte unterschreitet, kann dies ein Anzeichen für das Vorhandensein von Metallelementen 6 innerhalb der Körperregion R sein, wodurch eine entsprechende Anzeige an der Anzeigeeinheit 7 resultiert. Als Hautelektroden 13 kommen Klebeelektroden oder auch Metallelektroden in Frage. Diese können in einem Gehäuse, in dem die zumindest eine Magnetspule 2 zur Stimulation angeordnet ist, integriert sein oder auch in einem eigenen, von der Stimulationsvorrichtung 1 getrennten Gehäuse angeordnet sein.

Schließlich ist in Fig. 5 ein Blockschaltbild der magnetischen Stimulationsvorrichtung 1 mit einer Variante einer weiteren Detektionseinheit 5' in Form einer Messspule 19 wiedergegeben. Bei dieser Ausführungsvariante wird mit zumindest einer Messspule 19 in Art eines Metallsuchgeräts das Metallelement 6 in der Körperregion R erfasst und eine entsprechende Warnung an der Anzeigeeinheit 7 wiedergegeben und allenfalls ein Steuersignal an den Stimulator 3 geschickt. Die Messspule 19 unterscheidet sich vom Aufbau und von der Windungszahl deutlich von der zumindest einen Magnetspule 2 zur Erzeugung des Magnetfelds H, welches in der Körperregion R induziert werden soll. In dieser Ausführungsvariante sind die Komponenten der weiteren Detektionseinheit 5' und der Anzeigeeinheit 7 wieder in der magnetischen Stimulationsvorrichtung 1 angeordnet.

Die erfindungsgemäße Detektionseinheit 5 kann in einer magnetischen Stimulationsvorrichtung 1 integriert werden oder auch als Nachrüstbausatz in bestehende magnetische Stimulationsvorrichtungen 1 eingebaut werden. Dabei ist kein Eingriff in den Leistungsteil 4 des Stimulators 3 der Stimulationsvorrichtung 1 zwingend notwendig, sondern es kann die Reaktion der Magnetspule 2 auf den Stimulationsimpuls oder einen Testimpuls auch nur durch Messung der Spannung und des Stromes an den Zuleitungen zur Magnetspule 2 stattfinden. Zu diesem Zweck sind bloß entsprechende Leitungen zum Abgriff der Spannung an den Zuleitungen zur Magnetspule 2 und ein Stromwandler zur Erfassung des Stromes erforderlich.

Eine alternative Methode der Detektion von Metallelementen innerhalb des Magnetfelds einer Magnetspule über die Leistungsaufnahme der Magnetspule, kann auch durch indirekte Messung der Restspannung an einem Speicherkondensator, der vor jeder Impulsabgabe auf eine hohe Spannung aufgeladen wird und die notwendige Energie speichert, wie er für die Erzeugung der Stimulationsimpulse üblicher Weise zum Einsatz kommt, erfolgen. Durch den Einfluss von Metallelementen innerhalb des Magnetfelds der Magnetspule, ändert sich die Impedanz der Magnetspule und somit die Restspannung am Kondensator zur Erzeugung des Stimulationsimpulses nach Abgabe des Stimulationsimpulses. Diese letztgenannte Methode erfordert allerdings einen Eingriff in den Leistungsteil des Stimulators der Stimulationsvorrichtung.

## Patentansprüche

1. Vorrichtung (1) zur magnetischen Stimulation von Regionen (R) eines menschlichen oder tierischen Körpers, mit zumindest einer Magnetspule (2), welche mit einem Stimulator (3) verbunden ist, der einen Leistungsteil (4) zur Erzeugung an die zumindest eine Magnetspule (2) anzulegende elektrischer Impulse (I) aufweist, sodass das in der zumindest einen Magnetspule (2) erzeugte Magnetfeld (H) in der Körperregion (R) induzierbar ist, wobei eine Detektionseinheit (5) zur Detektion von Metallelementen (6) innerhalb der Körperregion (R), in welcher das Magnetfeld (H) induziert wird, vorgesehen ist, und die Detektionseinheit (5) mit einer Anzeigeeinheit (7) und dem Stimulator (3) verbunden ist, **dadurch gekennzeichnet, dass** eine Messeinrichtung (8) zur Erfassung der von der zumindest einen Magnetspule (2) aufgenommenen elektrischen Leistung (P) als Reaktion auf ein in die Magnetspule (2) eingespeistes Testsignal durch Messung von Betrag und Phase des Stroms und der Spannung an der Magnetspule (2) und eine Vergleichseinrichtung (9) zum Vergleich der aufgenommenen elektrischen Leistung (P) mit einem vorgegebenen Grenzwert (Pₘₐₓ), der bei einer vorausgehenden Kalibration eingestellt wird, vorgesehen ist, und die Vergleichseinrichtung (9) dazu ausgebildet ist, im Falle der Überschreitung des vorgegebenen Grenzwerts (Pₘₐₓ) den Stimulator (3) automatisch abzuschalten oder die Leistung des Stimulators (3) bzw. des Leistungsteils (4) des Stimulators (3) zu reduzieren, und dass zumindest eine weitere Detektionseinheit (5') zur Detektion von Metallelementen (6) innerhalb der Körperregion (R) vorgesehen ist, welche durch zumindest einen Ultraschallsender (10) und zumindest einen Ultraschallempfänger (11) und eine Auswerteeinheit (12) und bzw. oder durch zumindest zwei Hautelektroden (13) und eine Einrichtung (14) zur Messung der Gewebeimpedanz (Z) der jeweiligen Körperregion (R) gebildet ist.

2. Magnetische Stimulationsvorrichtung (1) nach Anspruch 1, **dadurch gekennzeichnet, dass** die weitere Detektionseinheit (5') durch zumindest eine Messspule (19) gebildet ist.

3. Magnetische Stimulationsvorrichtung (1) nach Anspruch 1 oder 2, **dadurch gekennzeichnet, dass** die Anzeigeeinheit (7) durch eine optische Anzeigeeinheit (15) und/oder eine akustische An-zeigeeinheit (16) und/oder einen mechanischen Schwingungserzeuger (17) gebildet ist.

4. Magnetische Stimulationsvorrichtung (1) nach einem der Ansprüche 1 bis 3, **dadurch gekennzeichnet, dass** die zumindest eine Magnetspule (2) in einem Gehäuse (18) angeordnet ist.

5. Magnetische Stimulationsvorrichtung (1) nach Anspruch 4, **dadurch gekennzeichnet, dass** die Detektionseinheit (5)und allenfalls die zumindest eine weitere Detektionseinheit (5') im Gehäuse (18) angeordnet ist.

## Claims

1. Device (1) for magnetically stimulating areas (R) of a human body or animal body, having at least one magnetic coil (2) which is connected to a stimulator (3) which has a power component (4) for generating electrical pulses (I) which are to be applied to the at least one magnetic coil (2), with the result that the magnetic field (H) which is generated in the at least one magnetic coil (2) can be induced in the body area (R), wherein a detection unit (5) is provided for detecting metal elements (6) within the body area (R) in which the magnetic field (H) is induced, and the detection unit (5) is connected to a display unit (7) and to the stimulator (3), **characterized in that** a measuring device (8) for measuring the electrical power (P) which is taken up by the at least one magnetic coil (2) is provided as a reaction to a test signal, fed into the magnetic coil (2), by measuring the absolute value and phase of the current and of the voltage at the magnetic coil (2), and a comparator device (9) for comparing the taken-up electrical power (P) with a predefined limiting value (Pₘₐₓ) which is set during a preceding calibration, and the comparator device (9) is designed to switch off the stimulator (3) automatically or to reduce the power of the stimulator (3) or of the power component (4) of the stimulator (3) in the event of the predefined limiting value (Pₘₐₓ) being exceeded, and **in that** at least one further detection unit (5') is provided for detecting metal elements (6) within the body area (R), which further detection unit 5') is formed by at least one ultrasonic transmitter (10) and at least one ultrasonic receiver (11), and an evaluation unit (12) and/or by means of at least two skin electrodes (13) and a device (14) for measuring the tissue impedance (Z) of the respective body area (R) .

2. Magnetic stimulation device (1) according to Claim 1, **characterized in that** the further detection unit (5') is formed by at least one measuring coil (19) .

3. Magnetic stimulation device (1) according to Claim 1 or 2, **characterized in that** the display unit (7) is formed by an optical display unit (15) and/or an acoustic display unit (16) and/or a mechanical vibration generator (17).

4. Magnetic stimulation device (1) according to one of Claims 1 to 3, **characterized in that** the at least one magnetic coil (2) is arranged in a housing (18).

5. Magnetic stimulation device (1) according to Claim 4, **characterized in that** the detection unit (5) and, if necessary, the at least one further detection unit (5') are arranged in the housing (18).

## Revendications

1. Dispositif (1) destiné à la stimulation magnétique de régions (R) d'un corps humain ou animal, avec au moins une bobine magnétique (2) qui est raccordée à un stimulateur (3) qui comporte une partie de puissance (4) destinée à la production d'impulsions (I) électriques devant être appliquées à l'au moins une bobine magnétique (2), de telle sorte que le champ magnétique (H) produit dans l'au moins une bobine magnétique (2) puisse être induit dans la région corporelle (R), une unité de détection (5) destinée à la détection d'éléments métalliques (6) à l'intérieur de la région corporelle (R) dans laquelle le champ magnétique (H) est induit étant prévue, et l'unité de détection (5) étant raccordée à une unité d'affichage (7) et au stimulateur (3), **caractérisé en ce qu'**il est prévu un équipement de mesure (8) destiné à la détection de la puissance (P) électrique absorbée par l'au moins une bobine magnétique (2) en tant que réaction à un signal test injecté dans la bobine magnétique (2) par la mesure de la valeur et de la phase du courant et de la tension sur la bobine magnétique (2), et un équipement de comparaison (9) destiné à la comparaison de la puissance (P) électrique absorbée à une valeur limite (Pₘₐₓ) spécifiée au préalable qui est réglée lors d'un étalonnage antérieur, et l'équipement de comparaison (9) est constitué pour, en cas de dépassement de la valeur limite (Pₘₐₓ) spécifiée au préalable, mettre automatiquement hors circuit le stimulateur (3) ou pour réduire automatiquement la puissance du stimulateur (3) ou plus précisément de la partie de puissance (4) du stimulateur (3), et **en ce qu'**au moins une autre unité de détection (5') est prévue pour la détection d'éléments métalliques (6) à l'intérieur de la région corporelle (R), laquelle est formée d'au moins un émetteur d'ultrasons (10) et d'au moins un récepteur d'ultrasons (11) et d'une unité d'analyse (12) et/ou d'au moins deux électrodes cutanées (13) et d'un équipement (14) destiné à la mesure de l'impédance tissulaire (Z) de la région corporelle (R) respective.

2. Dispositif (1) de stimulation magnétique selon la revendication 1, **caractérisé en ce que** l'autre unité de détection (5') est formée d'au moins une bobine de mesure (19).

3. Dispositif de simulation (1) magnétique selon la revendication 1 ou 2, **caractérisé en ce que** l'unité d'affichage (7) est formée d'une unité d'affichage (15) optique et/ou d'une unité d'affichage (16) acoustique et/ou d'un générateur de vibrations (17) mécanique.

4. Dispositif (1) de stimulation magnétique selon l'une des revendications 1 à 3, **caractérisé en ce que** l'au moins une bobine magnétique (2) est disposée dans un boîtier (18).

5. Dispositif (1) de stimulation magnétique selon la revendication 4, **caractérisé en ce que** l'unité de détection (5) et éventuellement l'au moins une autre unité de détection (5') sont disposées dans le boîtier (18).
